# EUROPÄISCHE PATENTSCHRIFT

(11) **EP 0 367 926 B1**
(45) Veröffentlichungstag und Bekanntmachung des Hinweises auf die Patenterteilung: **20.10.1993**
(21) Anmeldenummer: 89115806.5
(22) Anmeldetag: 28.08.1989
(51) Int. Cl.: A61K 7/13

(54) **Haarfärbe- und Haartönungsmittel**
Hair dyeing and tinting product
Composition pour la nuance des cheveux et leur coloration

(30) Priorität: 10.10.1988 DE 3834406
(43) Veröffentlichungstag der Anmeldung: 16.05.1990
(73) Patentinhaber: Blendax GmbH, D-55120 Mainz (DE)
(72) Erfinder: Schrader, Karlheinz, D-3454 Bevern (DE)
(74) Vertreter: TER MEER STEINMEISTER & PARTNER GbR

(56) Entgegenhaltungen:
- EP-A- 0 137 178
- FR-A- 2 113 845
- US-A- 4 329 335

## Beschreibung

Die vorliegende Erfindung betrifft ein Mittel zum Färben bzw. Tönen von menschlichem Haar, das optimale haarpflegende Eigenschaften mit guten galenischen Eigenschaften und einer besonders guten Hautverträglichkeit vereinigt.

Haarfärbemittel auf Basis sogenannter direktziehender Farbstoffe, die zur Farbentwicklung keiner oxidativen Einwirkung bedürfen, werden auch als Haartönungsmittel bezeichnet und sind seit langem bei den Verbraucherinnen bekannt und beliebt. Sie weisen gegenüber Haarfärbemitteln auf Basis sogenannter Oxidationsfarbstoffe den Vorteil einer schonenderen Behandlung des Haares auf und sind auch leichter aus dem Haar zu entfernen. Sie werden üblicherweise in einer aus Tensiden, insbesondere anionaktiven Tensiden, bestehenden Grundlage eingesetzt.

Ziel der Erfindung ist es, eine solche Zusammensetzung zu schaffen, die nicht nur gute anwendungstechnische Eigenschaften, sondern auch eine ausgezeichnete Haut- bzw. Schleimhautverträglichkeit aufweist und optimale haarkonditionierende Eigenschaften besitzt.

Diese Aufgabe wird dadurch gelöst, daß ein Haarfärbe- bzw. Haartönungsmittel auf Basis mindestens eines direktziehenden Farbstoffs in einer aus Tensiden und üblichen Zusatzstoffen bestehenden Grundlage zum Einsatz gelangt, wobei die Grundlage folgende Stoffe enthält:
2,5 bis 20, insbesondere 5 bis 10 Gew.-% mindestens eines amphoteren Tensids,
1 bis 10, vorzugsweise 2,5 bis 7,5 Gew.-% mindestens eines oberflächenaktiven Aminoxids;
1 bis 10, vorzugsweise 2,5 bis 5 Gew.-% mindestens eines nichtionischen Tensids.

Vorzugsweise enthält dieses Gemisch noch zur Verbesserung der Hautverträglichkeit und Konditionierwirkung zusätzlich 0,5 bis 5, vorzugsweise 1 bis 3 Gew.-%, eines Eiweißhydrolysates.

Aus der EP-A 46 543 sind bereits Mittel zum gleichzeitigen Färben bzw. Tönen, Waschen und Konditionieren von menschlichen Haaren bekannt. Diese bekannten Mittel enthalten jedoch obligatorisch Oxidationshaarfarbstoffe und beträchtliche Mengen an Fettalkoholethersulfaten, was zu Produkten führt, die hinsichtlich der milden Wirkung auf Haut und Haare nicht die Anforderungen erfüllen, wie sie in der Aufgabenstellung der vorliegenden Erfindung definiert sind.

Fettalkoholethersulfate sollen erfindungsgemäß, wenn überhaupt, nur in untergeordneten Mengen mitverwendet werden und bilden nicht die Grundlage des Tensidsystems; ebenso ist erfindungsgemäß die Verwendung von Oxidationshaarfarben nicht zwingend vorgesehen.

Geeignete amphotere Tenside sind insbesondere die bekannten oberflächenaktiven Betaine bzw. Sulfobetaine wie beispielsweise C 10 - C 18 - Alkylbetaine der allgemeinen Formel,

R - N⁺(R₁,R₂)-CH₂-COO⁻

wobei R einen langkettigen Alkylrest und die Reste R₁ und R₂ kurzkettige, gegebenenfalls hydroxysubstituierte Alkylgruppen wie Methyl-, Ethyl- oder Hydroxyethylgruppen bedeuten, wobei die langkettige Alkylgruppe auch eine Amidbindung enthalten kann, beispielsweise Kokosamidopropylbetain. Derartige Produkte sind unter dem Handelsnamen Tegobetain^{R} auf dem Markt.

Weitere geeignete Tenside vom Betain-Typ sind die unter dem Handelsnamen Miranol^{R} im Handel befindlichen Imidazolinderivate der allgemeinen Formel 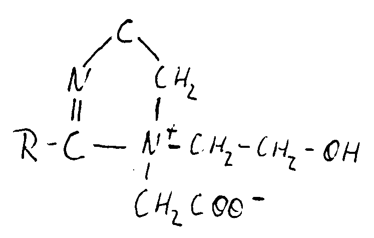
wobei R einen langkettigen Alkylrest bedeutet. Die Carboxylgruppe kann auch durch eine Sulfogruppe ersetzt sein.

Bei den Sulfobetainen ist die Carboxylgruppe der Betaine durch eine Sulfogruppe ersetzt.

Eine weitere Gruppe amphoterer Tenside sind langkettige N-Alkylaminocarbonsäuren, wie sie beispielsweise unter der Bezeichnung Deriphat^{R} im Handel sind.

Ein weiterer essentieller Bestandteil der erfindungsgemäßen Zusammensetzung sind oberflächenaktive Aminoxide.

Geeignete Aminoxide sind beispielsweise Trialkylaminoxide, die einen langkettigen Alkylrest mit 10 bis 20 C-Atomen und 2 kurzkettige Alkylreste, die gegebenenfalls hydroxysubstituiert sein können, enthalten, beispielsweise Lauryldimetylaminoxid oder Kokosdi(hydroxyethyl)aminoxid. Der langkettige Alkylrest kann auch durch Amidgruppen substituiert sein, beispielsweise ein C 7 - C 17 - Alkoylamino-3-dimethylaminopropan-3-N-oxid. Auch polyoxethylierte Aminoxide sind geeignet ebenso Aminoxide des Typs Diethylaminopropylpalmitamid-N-oxid, kokosamidopropyldimethylaminoxid, etc..

Eine Übersicht über geeignete Aminoxide findet sich beispielsweise bei L. Raphael Manufacturing, Cosmetics Aerosol News 50/ Nr. 5, 75 (1979).

Der dritte essentielle Bestandteil der erfindungsgemäßen Zusammensetzung sind nichtionische Tenside. Als solche sind insbesondere Polyoxyethylenfettsäureester geeignet, wie sie beispielsweise unter den Handelsnamen Arlatone^{R} oder Arlacel^{R} in den Handel gebracht werden. Geeignete Produkte sind beispielsweise Polyethylenglykolester mit hydriertem Rizinusöl, Polyoxyethylensorbitanfettsäureester, wie Polyethylenglykolsorbitanmonooleat, Polyoxyethylenmonostearat sowie auch Polyoxyethylenfettalkoholether, Polyethylenoxidester.

Derartige Produke sind hinreichend bekannt und in zahlreichen Handbüchern beschrieben.

Ein bevorzugter Bestandteil der erfindungsgemäßen Mischung sind Eiweißhydrolysate.

Derartige Hydrolysate sind an sich seit langem bekannt und werden in den verschiedensten kosmetischen Mitteln, insbesondere Shampoos und Schaumbädern, vorteilhaft eingesetzt, wobei die Molekulargewichte über einen weiten Bereich zwischen etwa 100 und 50.000, vorzugsweise 1.000 bis 5.000, schwanken können.

Bevorzugte Eiweißhydrolysate sind beispielsweise Seidenproteinhydrolysate sowie Kollagenhydrolysate im Molgewichtsbereich zwischen 1.000 und 1.500 sowie Keratinhydrolysate, die durch enzymkatalysierte Eiweißhydrolyse, beispielsweise mittels Trypsin, oder durch Säurehydrolyse erhalten worden sind und gegebenenfalls auch durch Umsetzung mit Glycidylalkylammoniumsalzen oder 3-Halo-2-hydroxypropyltrialkylammoniumsalzen quaternisiert sein können.

Eine Übersicht über die in kosmetischen Mitteln zum Einsatz gelangenden Eiweißhydrolysate findet sich beispielsweise bei A.L. Johnson, Drug, Cosmetic Industry 126/ Nr. 6 (1980), 36, sowie G.J. Brooks, Cosmetics & Toiletries 94/ Nr. 7 (1979), 82.

Die erfindungsgemäßen Zusammensetzungen können selbstverständlich alle für Haarfärbe- bzw. Haartönungsmittel üblichen und bekannten Zusätze enthalten. Insbesondere ist die Mitverwendung weiterer haarkonditionierender Substanzen, vorzugsweise von quaternisierten Polymeren, möglich, wie sie beispielhaft in der bereits erwähnten EP-A 46,543 beschrieben sind.

Bevorzugt werden dabei Substanzen vom Typ quaternärer Cellulosederivate wie "Polymer JR" sowie synthetische quaternäre Polymere wie beispielsweise die unter den Handelsnamen Merquat^{R} oder Polyquart^{R} vertriebenen Produkte. Die Einsatzmenge derartiger Stoffe beträgt, soweit vorhanden, etwa 0,2 bis 1,5 Gew.-%, bezogen auf die Gesamtzusammensetzung.

Zweckmäßig ist auch die Mitverwendung von Verdickungsmitteln, beispielsweise Fettsäuremono- und-dialkanolamiden, die auch nichtionische oberflächenaktive Eigenschaften aufweisen, deren Einsatz als Verdickungsmittel in den erfindungsgemäßen Zusammensetzungen jedoch zusätzlich zu dem Anteil des nichtionischen Tensids zu sehen ist, ebenso wie der eines weiteren bewährten nichtionischen oberflächenaktiven Verdickungsmittels, nämlich Estern polyoxethylierter Glycoside wie Polyethylenglykolmethylglykosidoleat.

Weitere geeignete Verdickungsmittel sind Polymere wie Polyacrylsäuresalze und Cellulosederivate.

Die Verdickungsmittel sind in der Regel in einer Menge zwischen etwa 0,5 und 5 Gew.-% der Gesamtzusammensetzung enthalten.

Geeignete Stabilisatoren sind insbesondere Polyethylenglykole mit mittleren Molekulargewichten zwischen etwa 300 und 1500, beispielsweise Polyglykol 400. Sie können in einer Menge zwischen etwa 1 und 5 Gew.-% der Gesamtzusammensetzung enthalten sein.

Die erfindungsgemäßen Zusammensetzungen enthalten weiterhin alle in solchen Mitteln üblichen Stoffe, wie Konservierungsmittel soweit erforderlich Neutralisierungsmittel, Parfumzusammensetzungen, etc..

Ein essentieller Bestandteil der erfindungsgemäßen Haarfärbe- und Haartönungsmittel sind natürlich die an sich bekannten direktziehenden Haarfarben.

Deren Auswahl und Konzentration ist selbstverständlich abhängig von der gewünschten Farbnuance, weshalb die folgende Aufzählung nur eine beispielhafte sein kann.

Besonders geeignet sind die sogenannten Arianor-Farbstoffe Basic Brown 17, C.I. (Colour Index)- No. 12,251; Basic Red 76, C.I.-No. 12,245; Basic Brown 16, C.I.- No. 12, 250; Basic Yellow 57, C.I.-No. 12,719 und Basic Blue 99, C.I.-No. 56,059 sowie weitere direktziehende Farbstoffe wie insbesondere Acid Yellow 1, C.I.-No. 10,316; Acid Yellow 9, C.I.-No. 13,015; Basic Violet C.I.-No. 45,170; Disperse Yellow 3, C.I.-No. 11,855; Basic Yellow 57, C.I.-No. 12,719; Disperse Yellow 1, C.I.-No. 10,345; Basic Violet 1, C.I.-No. 42,535, und Basic Violet 3, C.I.-No. 42,555.

Auch der Einsatz von Naturfarben wie Henna in den erfindungsgemäßen Zusammensetzungen ist möglich.

Die erfindungsgemäßen Haarfärbe- bzw. -tönungsmittel liegen vorzugsweise als Schaumaerosole, sogenannte Schaumtönungen oder Tönungsschäume, vor.

Zu diesem Zweck wird das Färbemittel auf wäßriger Basis mit einem Aerosoltreibmittel, vorzugsweise im Gewichtsverhältnis von 85 bis 97:3 bis 15, vermischt.

Bevorzugte Treibmittel sind dabei niedere Kohlenwasserstoffe wie Propan, Butan und Isobutan, wobei bei deren Verwendung ihr Anteil zwischen etwa 3 und 8 Gewichtsprozent der gesamten Schaumaerosolzusammensetzung beträgt; werden halogenierte Kohlenwasserstoffe eingesetzt, wird ein höherer Treibmittelanteil von etwa 7 bis 15 Gew.-% benötigt.

Auch der Einsatz von Dimethylether als Treibmittel ist möglich, wobei die Gewichtsanteile bei denen der niedrigen Kohlenwasserstoffe liegen.

Im folgenden werden einige Beispiele für erfindungsgemäße Zusammensetzungen gegeben:

### Beispiel 1

| | |
|---|---|
| Proteinhydrolysat | 2,00 (Gew.-%) |
| EDTA, Dinatriumsalz | 0,80 |
| Polyoxyethylen (120)-methylglucosiddioleat | 2,50 |
| Kokosamidpropylbetain | 5,00 |
| Kokosalkanolamid | 2,00 |
| Polyglycol | 3,00 |
| Kokosamidpropyldimethylaminoxid | 5,00 |
| Natriumpolyacrylat | 0,40 |
| Polyoxyethylensorbitanmonostearat | 3,50 |
| Konservierungsmittel | 0,20 |
| Parfum | 0,20 |
| Farbstoff C.I.-No. 12,719 | 0,02 |
| Farbstoff C.I.-No. 12,251 | 0,02 |
| Farbstoff C.I.-No. 12,250 | 0,04 |
| Farbstoff C.I.-No. 56,059 | 0,03 |
| Wasser | ad 100,00 |

95 Gew.-% dieser Mischung wurden mit 5 Gew.-% eines handelsüblichen Propan / Butan-Treibgasgemisches in einer Aerosoldose abgefüllt.

Bei der Anwendung des Schaumes wird ein hellblonder Farbton erzielt.

### Beispiel 2

| | |
|---|---|
| Seidenfibroinhydrolysat | 1,50 (Gew.-%) |
| Kokosdimethylaminoxid | 5,50 |
| Quaternärer Celluloseether | 1,00 |
| Polyethylenglykolester mit hydriertem Rizinusöl | 4,50 |
| Stearylamidopropylsulfobetain | 7,50 |
| Hydroxyethylcellulose | 0,25 |
| EDTA, Dinatriumsalz | 0,25 |
| Stearyltrimethylammoniumchlorid | 0,50 |
| Laurylmonoethanolamid | 2,00 |
| Konservierungsmittel | 0,20 |
| Parfum | 0,40 |
| Farbstoff C.I.-No. 12,719 | 0,03 |
| Farbstoff C.I.-No. 12,250 | 0,10 |
| Farbstoff C.I.-No. 12,245 | 0,05 |
| Farbstoff C.I.-No. 42,535 | 0,02 |
| Wasser | ad 100,00 |

88 Gew.-% dieser Zusammensetzung wurden mit 12 Gew.-% eines Treibmittelgemisches aus Dichlordifluormethan / Dichlortetrafluorethan (40:60) zur Herstellung eines Schaumaerosols abgefüllt.

Bei der Anwendung der Schaumtönung auf menschlichem Haar wird eine haselnußfarbige Tönung erhalten.

### Beispiel 3

| | |
|---|---|
| Glycerinmonostearat | 5,50 (Gew.-%) |
| Polyoxyethylenstearylether | 1,50 |
| C-20-C 22-Alkyltrimethyl.ammoniumchlorid | 0,50 |
| Kokosamidopropylsulfobetain | 6,00 |
| Lauryldimethylaminoxid | 5,00 |
| Harnstoffphosphat | 0,70 |
| Kollagenhydrolysat (MG 1.100) | 2,00 |
| EDTA, Tetranatriumsalz | 0,50 |
| Polyacrylsäure, Natriumsalz | 0,40 |
| Ethoxyliertes Methylglucosid | 2,00 |
| Konservierungsmittel | 0,20 |
| Parfum | 0,45 |
| Farbstoff C.I.-No. 12,250 | 0,07 |
| Farbstoff C.I.-No. 56,059 | 0,05 |
| Wasser | ad 100,00 |

95 Gew.-% dieser Zusammensetzung werden mit 5 Gew.-% Dimethylether in eine Aerosoldose abgefüllt.

Bei Anwendung dieses Tönungsschaumes wird eine mockkabraune Haarfärbung erhalten.

### Beispiel 4

| | |
|---|---|
| Polyoxyethylensorbitanmonoleat | 3,50 (Gew.-%) |
| Stearylamidopropyldimethylaminoxid | 3,00 |
| Polyethylenimin (MG 600.000) | 0,60 |
| Kokosamidopropylbetain | 2,80 |
| Seidenproteinhydrolysat | 0,85 |
| Polyglykol 400 | 3,00 |
| EDTA, Trinatriumsalz | 0,80 |
| Kokosfettsäuremonoethanolamid | 2,50 |
| Konservierungsmittel | 0,20 |
| Parfum | 0,40 |
| Farbstoff C.I.-No. 12,251 | 0,11 |
| Farbstoff C.I.-No. 56,059 | 0,60 |
| Farbstoff C.I.-No. 12,250 | 0,19 |
| Farbstoff C.I.-No. 12,245 | 0,10 |
| Wasser | ad 100,00 |

95 Gew-% dieser Zusammensetzung werden mit 5 Gew.-% eines handelsüblichen Propan / Butan / Isobutan-Treibmittel-Gemisches in eine Aerosoldose abgefüllt.

Bei der Anwendung dieses Tönungsschaumes auf menschliches Haar wurde eine Schwarzfärbung erhalten.

## Patentansprüche

1. Haarfärbe- und Haartönungsmittel, enthalten mindestens einen direktziehenden Farbstoff in einer aus Tensiden und üblichen Zusatzstoffen bestehenden Grundlage auf wäßriger Basis, dadurch gekennzeichnet, daß die Grundlage folgende Stoffe in den angegebenen Mengen enthält:
2,5 bis 20 Gew.-% mindestens eines amphoteren Tensids;
1 bis 10 Gew.-% mindestens eines oberflächenaktiven Aminoxids;
1 bis 10 Gew.-% mindestens eines nichtionischen Tensids

2. Mittel nach Anspruch 1, dadurch gekennzeichnet, daß es 5 bis 10 Gew.-% mindestens eines amphoteren Tensids enthält.

3. Mittel nach Anspruch 1 oder 2, dadurch gekennzeichnet, daß es als amphoteres Tensid mindestens ein oberflächenaktives Betain oder Sulfobetain enthält.

4. Mittel nach eindem der Ansprüche 1 bis 3, dadurch gekennzeichnet, daß es 2,5 bis 7,5 Gew.-% mindestens eines oberflächenaktiven Aminoxids enthält.

5. Mittel nach einem der Ansprüche 1 bis 4, dadurch gekennzeichnet, daß es 2,5 bis 5 Gew.-% mindestens eines nichtionischen Tensids enthält.

6. Mittel nach einem oder mehreren der Ansprüche 1 bis 5 dadurch gekennzeichnet, daß es als nichtionisches Tensid einen Polyethylenoxidester enthält.

7. Mittel nach einem oder mehreren der Ansprüche 1 bis 6, dadurch gekennzeichnet, daß es als oberflächenaktives Aminoxid ein Kokosamidopropyldimethylaminoxid enthält.

8. Mittel nach einem oder mehreren der Ansprüche 1 bis 7, dadurch gekennzeichnet, daß es 0,5 bis 5 Gew.-% mindestens eines Eiweißhydrolysats enthält.

9. Mittel nach einem der Ansprüche 1 bis 8, dadurch gekennzeichnet, daß es 3 bis 15 Gew.-% eines Aerosoltreibmittels enthält und als Aerosolschaum vorliegt.

## Claims

1. Hair-colouring and hair-tinting compositions which contain at least one direct dye in a base which consists of surfactants and customary additives and is based on water, characterised in that the base contains the following substances in the amounts indicated:
2.5 to 20% by weight of at least one amphoteric surfactant;
1 to 10% by weight of at least one surface-active amine oxide;
1 to 10% by weight of at least one non-ionic surfactant.

2. Composition according to Claim 1, characterised in that it contains 5 to 10% by weight of at least one amphoteric surfactant.

3. Composition according to Claim 1 or 2, characterised in that as amphoteric surfactant it contains at least one surface-active betain or sulphobetain.

4. Composition according to one of Claims 1 to 3, characterised in that it contains 2.5 to 7.5% by weight of at least one surface-active amine oxide.

5. Composition according to one of Claims 1 to 4, characterised in that it contains 2.5 to 5% by weight of at least one non-ionic surfactant.

6. Composition according to one or more of Claims 1 to 5, characterised in that as non-ionic surfactant it contains a polyethylene oxide ester.

7. Composition according to one or more of Claims 1 to 6, characterised in that as surface-active amine oxide it contains a coconutamidopropyldimethylamine oxide.

8. Composition according to one or more of Claims 1 to 7, characterised in that it contains 0.5 to 5% by weight of at least one protein hydrolysate.

9. Composition according to one of Claims 1 to 8, characterised in that it contains 3 to 15% by weight of an aerosol propellant and is present as an aerosol foam.

## Revendications

1. Agent de teinture et de nuançage de cheveux contenant au moins un colorant à action directe dans une base aqueuse constituée d'agents tensio-actifs et des additifs usuels, caractérise en ce que la base contient les substances suivantes dans les quantités indiquées :
2,5 à 20 % en poids au moins d'un agent tensio-actif amphotère,
1 à 10 % en poids au moins d'un amino-oxyde tensio-actif,
1 à 10 % en poids au moins d'un agent tensio-actif non ionique.

2. Agent selon la revendication 1, caractérisé en ce qu'il contient 5 à 10 % en poids au moins d'un agent tensio-actif amphotère.

3. Agent selon la revendication 1 ou 2, caractérisé en ce qu'il contient comme agent tensio-actif amphotère au moins une bétaïne ou une sulfobétaïne tensio-active.

4. Agent selon l'une quelconque des revendications 1 à 3, caractérisé en ce qu'il contient 2,5 à 7,5 % en poids au moins d'un amino-oxyde tensio-actif.

5. Agent selon l'une quelconque des revendications 1 à 4, caractérisé en ce qu'il contient 2,5 à 5 % en poids au moins d'un agent tensio-actif non ionique.

6. Agent selon une ou plusieurs des revendications 1 à 5, caractérisé en ce qu'il contient comme agent tensio-actif non ionique un ester d'oxyde de polyéthylène.

7. Agent selon une ou plusieurs des revendications 1 à 6, caractérisé en ce qu'il contient comme amino-oxyde tensio-actif un amidopropyldiméthylamino-oxyde de coco.

8. Agent selon une ou plusieurs des revendications 1 à 7, caractérisé en ce qu'il contient 0,5 à 5 % en poids au moins d'un hydrolysat d'albumen.

9. Agent selon l'une quelconque des revendications 1 à 8, caractérisé en ce qu'il contient 3 à 15 % en poids d'un propulseur d'aérosol et se présente sous la forme d'une mousse d'aérosol.
